# EUROPEAN PATENT APPLICATION

(11) **EP 1 745 815 A1**
(43) Date of publication of application: **24.01.2007**
(21) Application number: 06076374.5
(22) Date of filing: 07.07.2006
(51) Int. Cl.: A61M 5/46, A44B 21/00

(54) **Side loading needle depth stop**

(30) Priority: 18.07.2005 US 700239 P
(71) Applicant: Atrion Medical Products, Inc., Arab, Alabama 35016 (US)
(72) Inventor: Kanner, Rowland W., Guntersville Alabama 35976 (US); Young, Larry Lee, Arab Alabama 35016 (US)
(74) Representative: Long, Edward Anthony

(57) **Abstract**

A needle depth stop (20, 120) which is side loading and configured to accommodate a wide range of needle diameters. The needle depth stop (20, 120) includes jaws (36, 38, 136, 138) which are biased closed and indexed into place by a spring member (26, 126), such as a "C" ring. However, the jaws (36, 38, 136, 138) can be pivoted open for receiving a needle (40) by pinching together two finger grip portions (32, 34, 132, 134), the opening force deceasing at a decreasing rate as a result of the pivot (99) translating toward the springs load center. The needle depth stop (20, 120) preferably provides approximately one pound of resistance to longitudinal displacement along the needle, and only one hand is required to position and lock the needle depth stop (20, 120) into place. In order to accommodate a wide range of needle diameters, the jaws (36, 38, 136, 138) include a plurality of grooves, each configured to accommodate a different range of needle diameters.

## Description

### RELATED APPLICATION (PRIORITY CLAIM)

This application claims the benefit of U.S. provisional application Serial No. 60/700,239, filed July 18, 2005, which is hereby incorporated herein by reference in its entirety.

### BACKGROUND

The present invention generally relates to needle depth stop devices, and more specifically relates to a side loading needle depth stop.

Some current commercially available needle depth stops include threaded collet chuck type devices that employ either rigid grasping collet members or elastomeric grasping elements that displace inward when the body and cap members are screwed together. An example of such a needle depth stop is shown in United States Patent No. 4,710,171.

Other depth stop mechanisms, such as the depth stop disclosed in United States Patent No. 1,436,707 and shown in Figure 1 of the present application, employ a bent spring clip having apertures through which a needle may be inserted when the clip is deformed. The apertures are sized and positioned in a manner on the spring clip such that when sighted along their common center axis, the apertures present an elliptical target to the needle whose minor axis is less than the needle's diameter. User deformation of the spring clip presents the apertures with a smaller angle between their planes allowing a needle to pass through, and relaxation of this deformation causes the apertures to bite into the needle. Because these stops must subject needles to a bending force in order to hold, they become less suitable at fine needle sizes whereby a depth stop that has been miniaturized to prevent bending of a fine needle provides insufficient grip and is hard to manipulate with gloved hands. These depth stops can also scratch and nick the needle's surface making tissue penetration more difficult.

Still other types of needle depth stop devices, such as is disclosed in United States Patent No. 3,477,437 and shown in Figure 2 of the present application, are coil spring type depth stops which have coil spacing which is close to the diameter of the needle which is desired to be retained. Typically, the retention forces of such devices are generally low, and bumping the spring from the wrong side can turn the compressive force that retains the needle into a strong ejection force causing the device to propel or eject the needle. Additionally, the needle depth stop is designed such that loading requires one to wedge the needle into place, thus subjecting the needle to bending forces which may be potentially detrimental, especially to very fine needles. Still further, the design provides that the spring does not sit on the needle squarely, preferring instead to sit at an angle approximately equal to the cant of the spring's coils.

Tightly wound springs, such as is shown in United States Patent No. 5,217,438 and shown in Figure 3 of the present application, that are made with an inside diameter slightly smaller than the axially inserted needle they are to retain can be used as depth stops. The inside diameter can be caused to open by creating an unwinding or uncoiling force as demonstrated in the '438 patent. Since the coil spring wire must be significantly less than the needle diameter it is used upon, this design becomes difficult to manufacture with the precision necessary for use on fine diameter needles. Needle depth stops of this design must be miniaturized to make the concept work on small diameter needles, which results in low retention forces and difficult manipulation with gloved hands.

The simplest depth stop consists of a disk of molded plastic having a slightly undersized hole through which the needle is passed. A stop such as this is available from Cook Inc. in Bloomington, Indiana, and is presently marketed as their "Centimeasure™ Biopsy Depth Marker". Due to the function and design of the device, retention force and reliability depends upon the plastic resin's compression set characteristic.

The above depth stops are listed in approximate order of their ability to be used on a range of needle gauges. Devices near the top tend to have more tolerance for use with a range of needle diameters. Devices near the bottom tend to be more single needle diameter specific.

An ideal needle depth stop would be a) a single device that can be used on a broad range of needle diameters, b) one that sits squarely to the needle, c) one that can be easily loaded from the needle's side instead of passing over the sharp tip, d) one that does not require great manufacturing precision or depend heavily upon a plastic resin's compression set character, e) one that retains itself passively instead of requiring tightening into place, f) one that only requires a single hand to apply or move, g) one that is inexpensive to manufacture, h) one that is no larger in diameter than a penny, i) one that delivers reduced compression force upon small diameter needles, and j) one that has an actuating force that increases at a decreasing rate.

Compared against the ideal product, all concepts discussed above fall short. Each depth stop type must be loaded by passing the sharp needle through an aperture, series of apertures or along a bore. This subjects the sharp needle tip and cutting edges to potential damage from inadvertent contact with the stop during the loading procedure. Nicked or dulled needles are undesirable because they do not perform properly, as they tend to tear tissue and be painful to the patient. A more serious risk is that a health care worker may become impaled or nicked by a sharp needle while installing a depth stop. With needle handling safety being a major problem and concern in today's healthcare environment, in order to prevent needle sticks, workers are instructed to never place themselves in front of sharp, potentially contaminated needles. Side loading onto a needle is therefore essential for any user-installed needle depth stop to succeed in today's health care environment. Additionally, needle depth stops such as are shown in United States Patent Nos. 4,710,171 and 3,477,437 require users to utilize two hands to apply and secure them, while needle depth stops such as is shown in United States Patent No. 1,436,707 and such as Cook Inc.'s "Centimeasure™ Biopsy Depth Marker" are effectively too small to handle and provide unacceptable retention forces when employed on fine needles.

### OBJECTS AND SUMMARY

An object of an embodiment of the present invention is to provide a needle depth stop that can be used on a broad range of needle diameters.

Another object of an embodiment of the present invention is to provide a needle depth stop that is configured to sit squarely to the needle.

Still another object of an embodiment of the present invention is to provide a needle depth stop that can be easily loaded from the needle's side instead of passing over the sharp tip.

Yet another object of an embodiment of the present invention is to provide a needle depth stop that does not require great manufacturing precision or depend heavily upon a plastic resin's compression set character.

Still yet another object of an embodiment of the present invention is to provide a needle depth stop that retains itself passively instead of requiring tightening into place.

Still yet another object of an embodiment of the present invention is to provide a needle depth stop that only requires a single hand to apply or move.

Still yet another object of an embodiment of the present invention is to provide a needle depth stop that is inexpensive to manufacture.

Still yet another object of an embodiment of the present invention is to provide a needle depth stop that is no larger in diameter than a penny.

Still yet another object of an embodiment of the present invention is to provide a needle depth stop that delivers reduced compression force upon small diameter needles.

Still yet another object of an embodiment of the present invention is to provide a spring-loaded needle depth stop having an actuating force that increases at a decreasing rate.

Briefly, and in accordance with at least one of the foregoing objects, an embodiment of the present invention provides a needle depth stop which is side loading, having jaws which can be opened by pinching together two finger grip portions. The jaws of the needle depth stop are generally biased closed by a spring member, but are openable by pinching together the two finger grip portions. Preferably, the jaws include a plurality of groove pairs, wherein each pair of grooves is configured to accommodate a different range of needles. Alternatively, the jaws may consists or a plurality of mating serrations or teeth. Preferably, the device is configured to work well on a wide range of needle sizes, such as needles ranging from 16 gauge (.065") through 25 gauge (.019"), a common range of sizes used for biopsy and depth/location marking With regard to the means for biasing the jaws closed, preferably a "C" ring is engaged with the jaws, and biases the jaws toward each other. The needle depth stop preferably provides approximately one pound of resistance to longitudinal displacement along the needle, and only one hand is required to position and lock the needle depth stop into place. The needle depth stop is also simple and inexpensive to manufacture, being composed of two "two plate" (simplest mold type) molded parts, indexed by and retained together with an inexpensive, commercially available stainless steel spring form derived from a commercially available stainless steel "C" ring. The "C" ring provides the essential compressive force for depth stop retention on a needle. Preferably, the pivot location of the depth stop is about 38% of the circle radius away from center, as this location strikes a reasonable balance between opening force and pivot stability.

Accommodation for a broad range of needles is achieved through a combination of the spring loaded jaw travel and a series of three decreasingly sized, opposed pairs of semicircular grooves in the needle stop's jaw halves or intermeshed jaw serrations. Preferably, the resulting diameter the grooves describe when the jaws are aligned parallel to one another is slightly larger than the smallest needle intended to be clamped within that groove. Preferably, the largest groove is situated closest to the jaw opening to reduce the degrees of jaw opening necessary for loading. The intermeshed jaw serrations are aligned parallel to one another and are preferably sized to cradle a retained needle within their "V"-shaped groove form, the needle being retained in place by the opposed intermeshing tooth form which provides a third point of contact. Preferably the largest "V"-shaped grooves and intermeshing teeth are situated closest to the jaw opening to reduce the degrees of jaw opening.

### BRIEF DESCRIPTION OF THE DRAWINGS

The organization and manner of the structure and operation of the invention, together with further objects and advantages thereof, may best be understood by reference to the following description, taken in connection with the accompanying drawings, wherein:

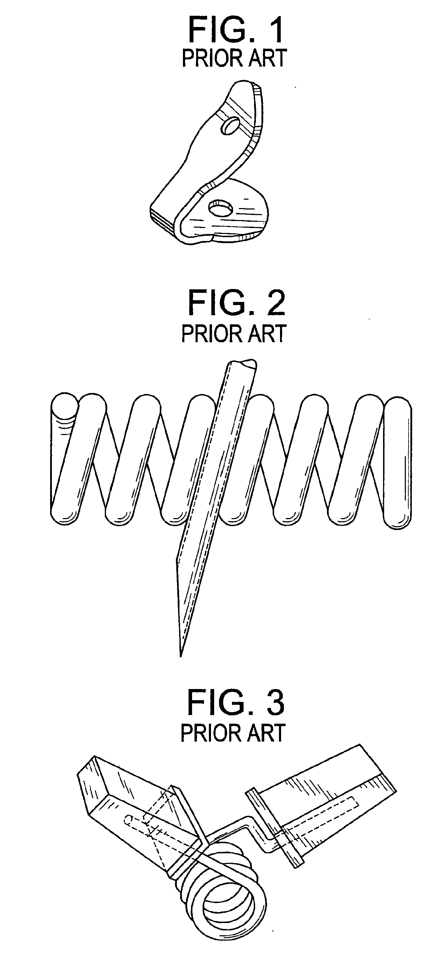

Figures 1 through 3 show prior art needle depth stops;
Figures 4 through 11 are three-dimensional illustrations of a needle depth stop which is in accordance with an embodiment of the present invention;
Figure 12 is a sketch which shows jaws of the device open to accept side insertion a needle, such as a 16 gauge needle (.057 inches in diameter);
Figures 13-17 are line drawing views of the needle depth stop shown in Figures 4-12;
Figures 18 and 19 show a "C" ring of the needle depth stop secured to a shaft;
Figures 20-22 illustrate a needle depth stop which is in accordance with an alternative embodiment of the present invention;
Figure 23 provides an exploded perspective view of the needle depth stop of Figures 20-22, with a spring member omitted for clarity;
Figures 24-30 show the jaws of the needle depth stop of Figures 20-22 gripping a different-sized Gauge needle;
Figure 31 shows the needle depth stop of Figures 4-17, shown in the closed position; and
Figure 32 shows the needle depth stop of Figures 4-17, shown in the open position.

### DESCRIPTION OF EMBODIMENTS OF THE INVENTION

While the invention may be susceptible to embodiment in different forms, there are shown in the drawings, and herein will be described in detail, specific embodiments of the invention. The present disclosure is to be considered an example of the principles of the invention, and is not intended to limit the invention to that which is illustrated and described herein.

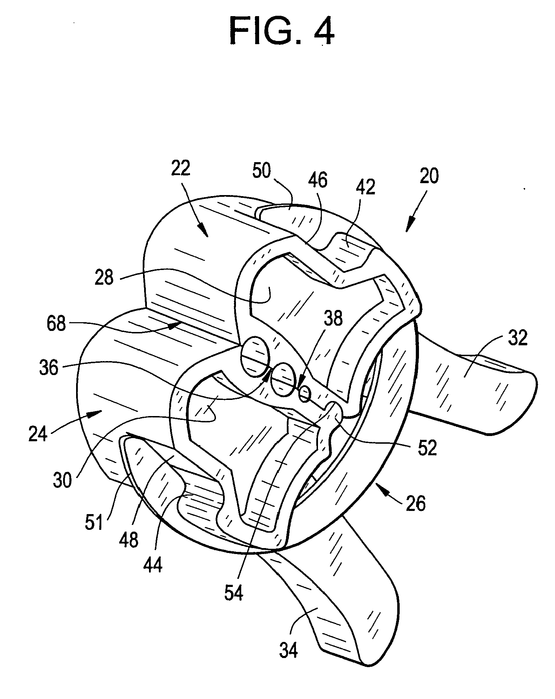

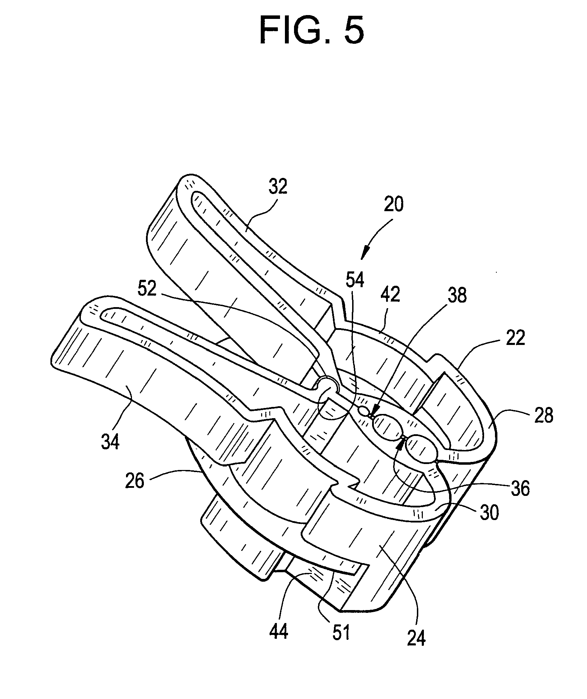

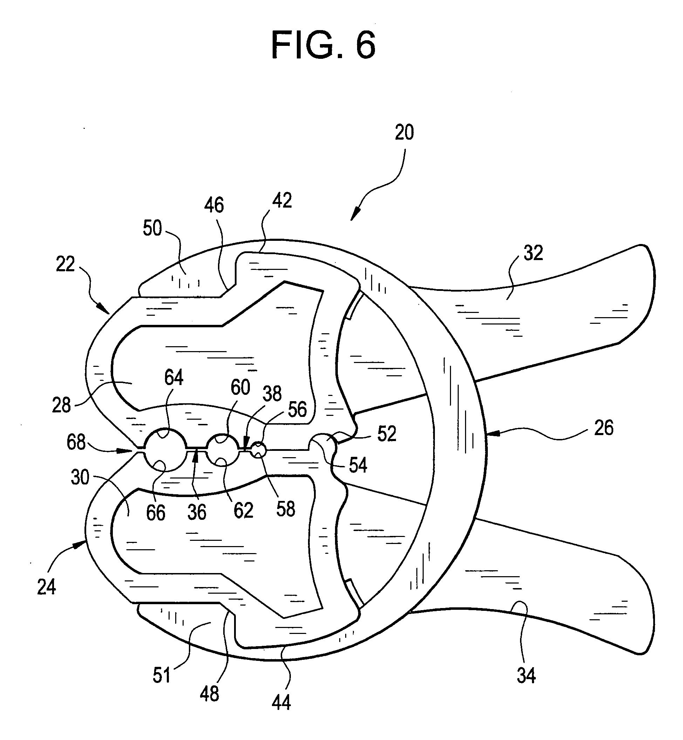

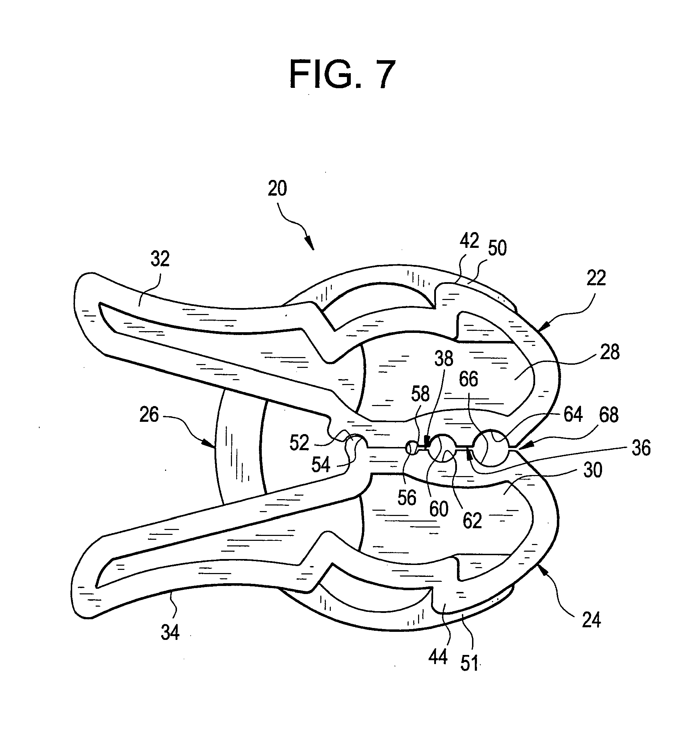

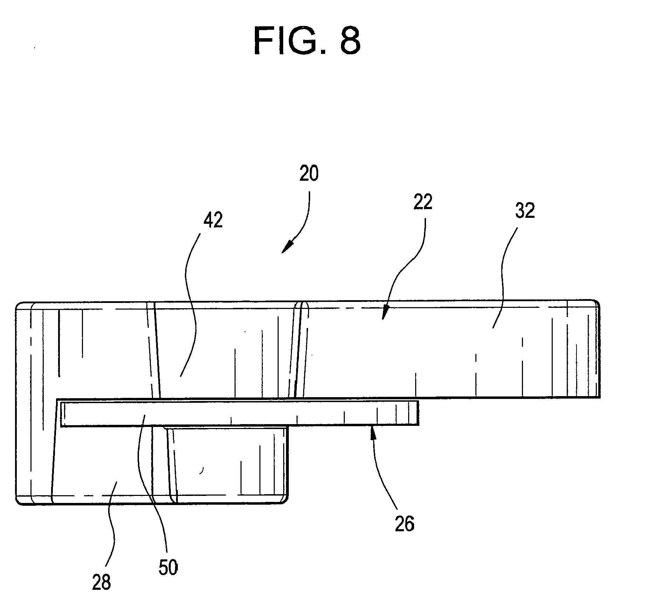

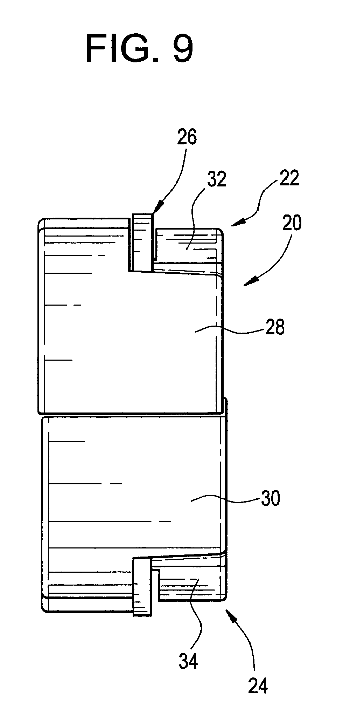

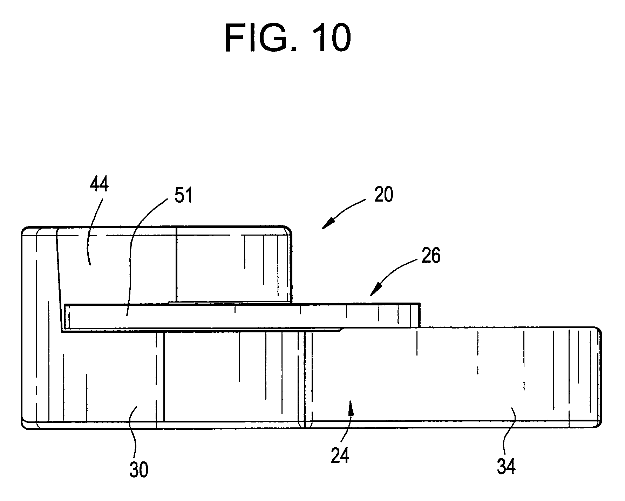

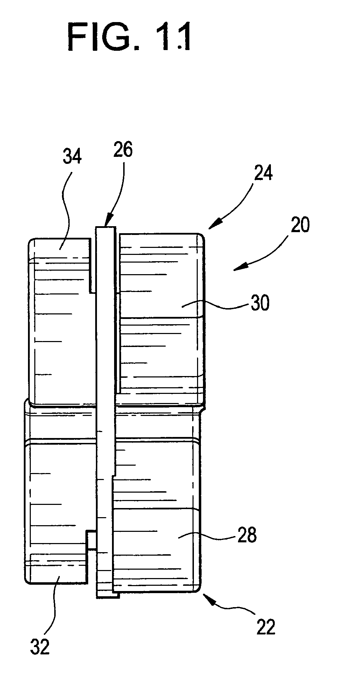

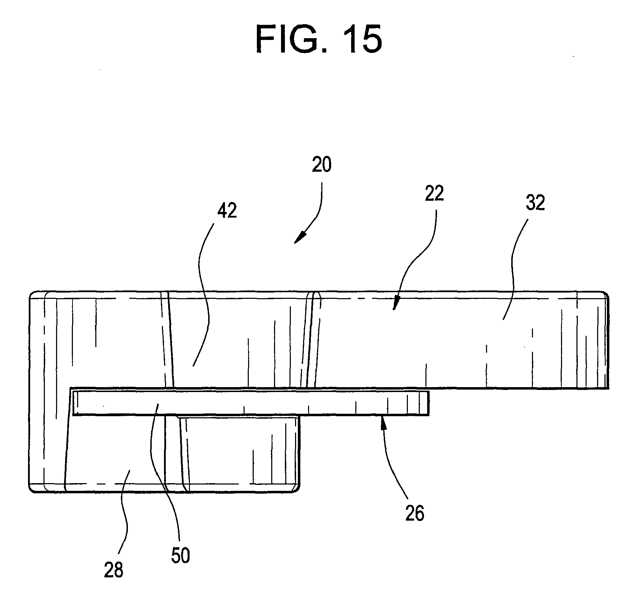

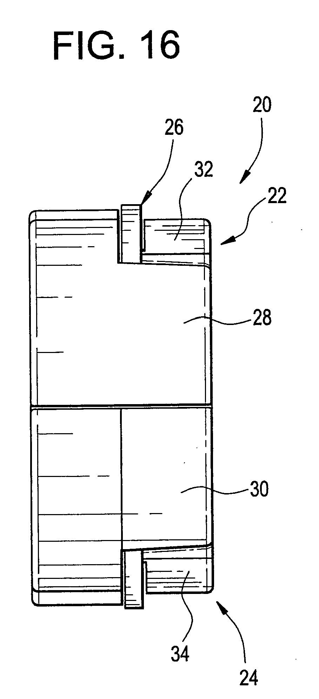

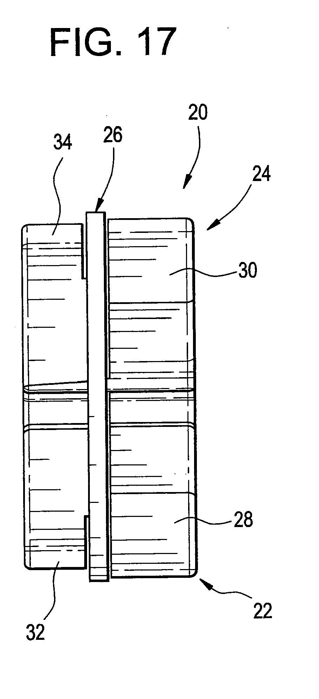

Figures 4-17 illustrate a needle depth stop 20 which is in accordance with an embodiment of the present invention. The needle depth stop 20 is configured for side loading onto a needle, instead of having to be passed over the sharp tip of the needle. The needle depth stop is configured to be operable using a single hand, and is configured to be used with a wide range of needle diameters. The depth stop is configured to sit squarely on a needle, and does not require great manufacturing precision or depend heavily upon a plastic resin's compression set character. The depth stop retains itself passively instead of requiring tightening into place, is inexpensive to manufacture and preferably is no larger in diameter than a penny.

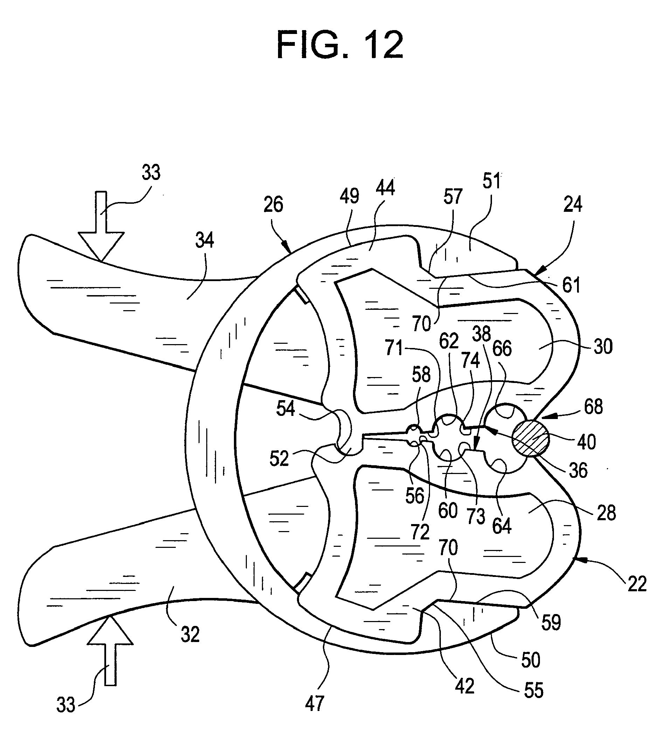

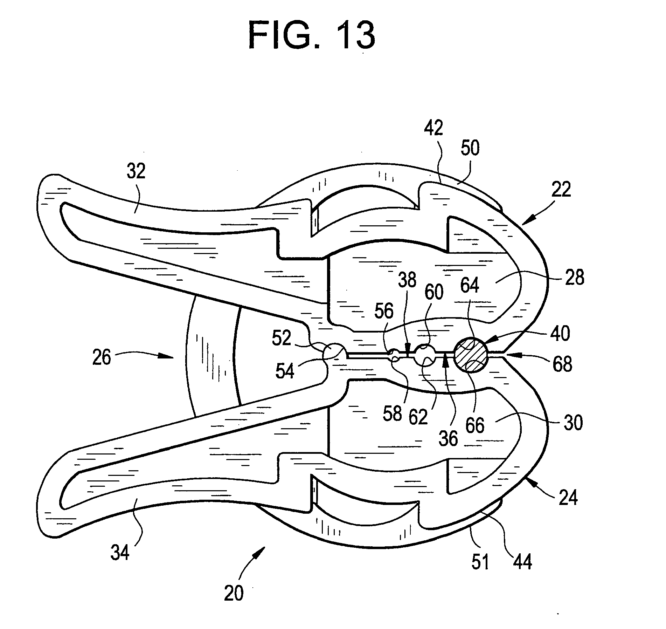

The needle depth stop 20 preferably consists of two body portions 22, 24 which are pivotably indexed together against longitudinal movement relative to each other and are indexed against axial motion by a spring member 26, such as a "C" ring, which engages the two body portions 22, 24 and biases them together. Preferably, each body portion 22, 24 is formed of plastic and includes a jaw portion 28, 30 and a finger grip portion 32, 34 which extends from the jaw portion 28, 30. The jaw portions 28, 30 work to come together to provide a set of jaws 36, 38 which are configured to grip a needle 40. The outer surface 42, 44 of each body portion 22, 24 also includes a recess 46, 48 which includes a groove 47, 49, a step 55, 57 and a pocket 59, 61. Each of the grooves 47, 49 receives a portion of the "C" ring 26, proximate the ends 50, 51 of the "C" ring 26, and each of the pockets 59, 61 receives a corresponding projection 70 at the end 50, 51 of the "C" ring 26. The needle depth stop 20 can be opened using one hand, specifically a thumb and index finger, on the finger grip portions 32, 34. Specifically, pressing the finger grip portions 32, 34 together (the pressing is represented by arrows 33 in Figure 12) causes the jaws 36, 38 to pivot open as shown in Figure 12, so that a needle 40 can be loaded from the side of the device 20, past an opening 68, in between the jaws 36, 38. Then, the finger grip portions 32, 34 can be released, causing the needle depth stop 20 to effectively lock in place on the needle 40, as shown in Figure 13.

Preferably, the device 20 is configured to work well on ten needle sizes ranging from 16 gauge (.065") through 25 gauge (.019"), a common range of sizes used for biopsy and depth/location marking. Preferably, the needle depth stop 20 provides approximately one pound of resistance to longitudinal displacement along the needle 40, and only one hand is required to operate the fmger grip portions 32, 34 and position and lock the needle depth stop 20 into place. As such, the needle depth stop 20 provides that there is never need for a user to reach in front of a sharp needle tip in order to install the depth stop 20. While the needle depth stop 20 can be configured to accommodate a range of needles from 16 gauge to 25 gauge, as discussed above, the same basic design can be scaled up or down for use in higher or lower size groupings of needles. Regardless, the needle depth stop 20 provides a user friendly device that holds well and does not dull, scratch, nick or deform fine needles.

The needle depth stop 20 not only is preferably usable over a large range of needle sizes, but is also simple and inexpensive to manufacture. These goals are accomplished by providing that each of the body portions 22, 24 is a "two plate" (simplest mold type) molded part, and that they are indexed by and retained together with an inexpensive, commercially available stainless steel spring form derived from a commercially available stainless steel "C" ring 26. The "C" ring 26 provides the essential compressive force for depth stop retention on a needle. Such a "C' ring is presently commercially available from Rotor Clip Co. or Truarc Co. Specifically, a "C" ring designed for a 9/16 inch (0.562 inches) shaft can be used.

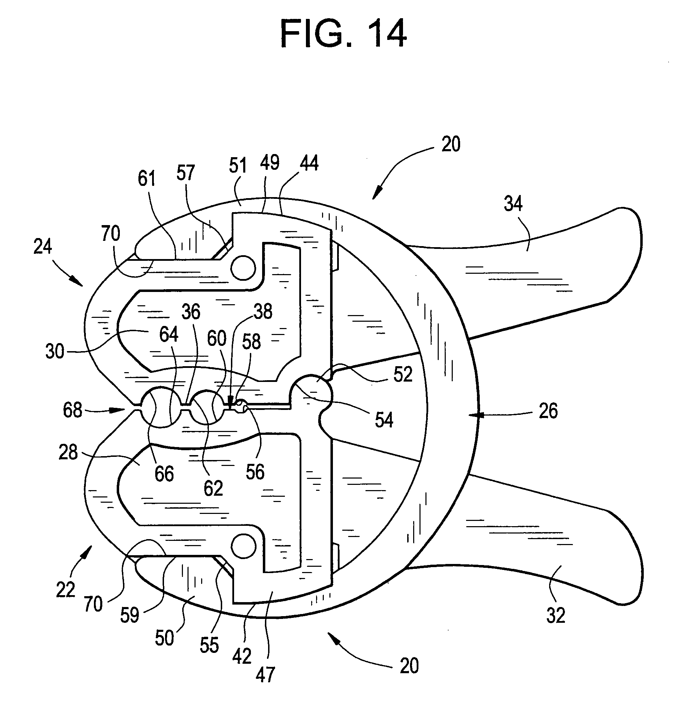

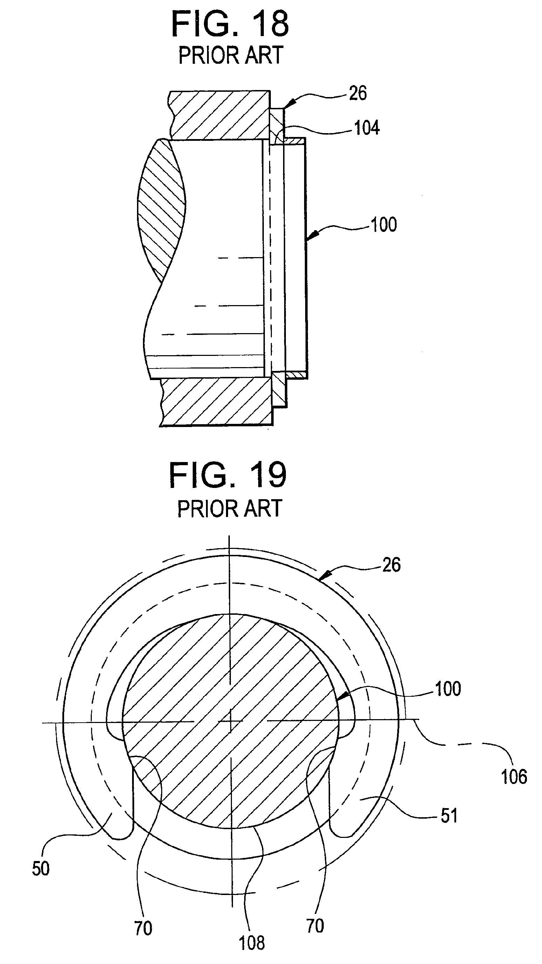

As shown in Figures 18 and 19, when the "C" ring is secured on a shaft 100 (which is the normal function of the "C" ring), the ring's interior surface 102 abuts a groove 104 on the shaft 100, and inward projections 70 at the ends 50, 51 of the "C" ring 26 travel past the shaft center 106 and grip on a back side 108 of the shaft 100, thereby securing the "C" ring 26 in place on the shaft 100. On the needle depth stop 20, the inward projections 70 on the ends 50, 51 of the "C" ring 26 pocket into, and reside in, a recess 46, 48 which is provided on the exterior surface 42, 44 of the body portions 22, 24, as shown in the drawings. Specifically, as discussed above, the cored out clearance 46, 48 includes a groove 47, 49, a step 55, 57 and a pocket 59, 61. Each of the grooves 47, 49 receive a portion of the "C" ring 26, proximate the ends 50, 51 of the "C" ring 26, and each of the pockets 59, 61 receives a corresponding projection 70 at the end 50, 51 of the "C" ring 26. The grooves 47, 49 are not as deep as the pocket 59, 61 (see Figure 14, for example), and a step 55, 57 is provided therebetween. As such, the ring 26 traverses when pushed into place from the rear of the assembly, and the projections 70 on the "C" ring 26 hook into place in the pockets 59, 61. Additionally, in order to harness the pure spring force, the needle depth stop 20 provides that, when the jaws 36, 38 are spread open as shown in Figure 12, only the projections 70 on the end of the "C" ring 26 are beared upon. As such, the steps 55, 57 (see Figure 14, for example) in the recesses 46, 48 do need to be sufficient to prevent the projections 70 from stepping out of the assembly when the jaws 36, 38 are spread.

While integral bending "hinge" members or integral spring members can be provided as being molded into the device shape, as shown in Figures 4-17, preferably the needle depth stop 20 utilizes a stainless steel "C" ring spring 26 and an unlinked hinge in the form of a molded pin and groove type pivot arrangement for connecting the two molded parts (i.e., the two body portions 22, 24). With regard to the pin and groove configuration which indexes the two body portions 22, 24 together, in Figures 4-17, reference numeral 52 is used to indicate the pin and reference numeral 54 is used to indicate the groove of the configuration. Preferably, the hinge indexing the body portions 22, 24 together is free to separate when needles are inserted into the jaw opening portion closest to the hinge.

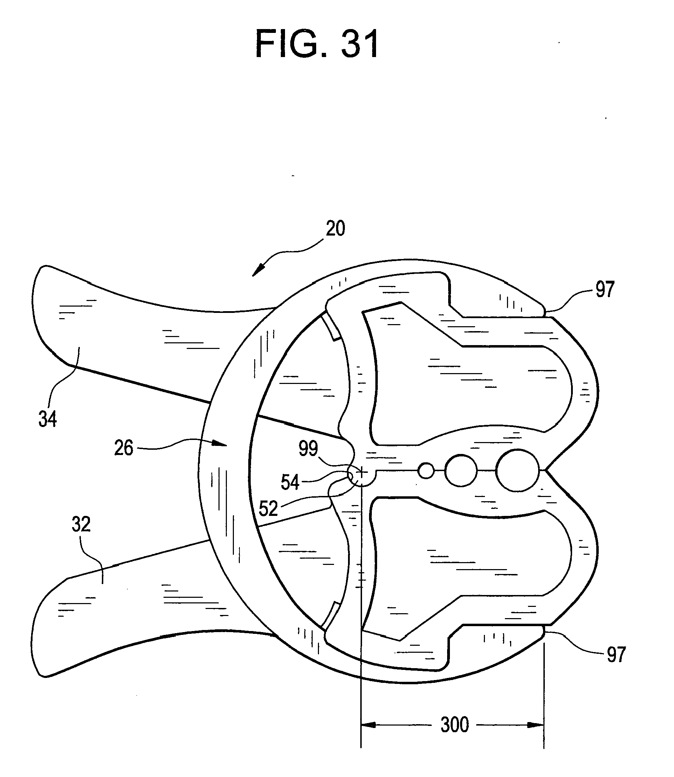

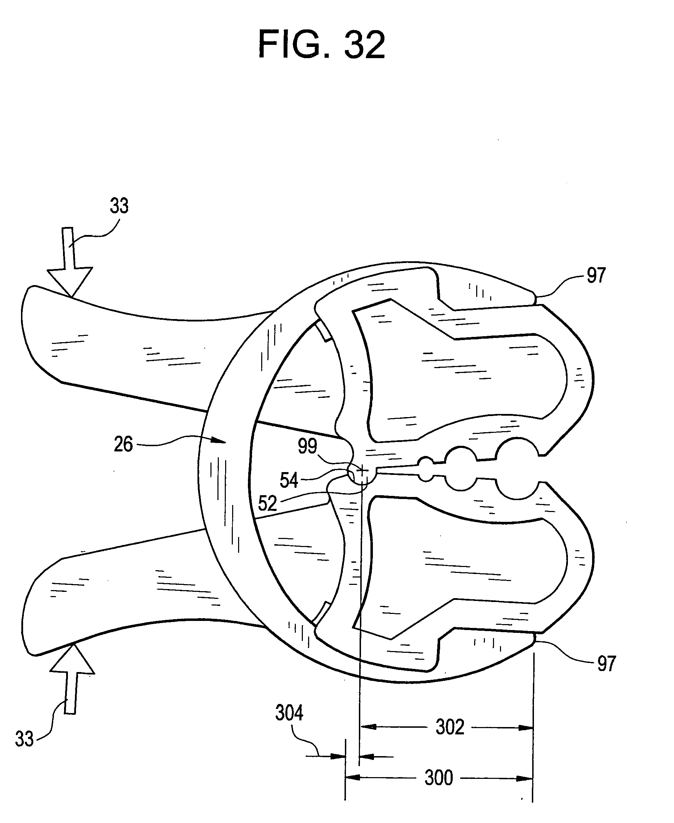

For size and mechanical advantage, the best location for this pivot is between the center of the device and an imaginary concentric circle tangent to the inside of the installed spring clip 26. Moving the pivot toward the device center creates a compound action that reduces load on the pivot while yielding a mechanical advantage to the user, resulting from a dynamic shift of the pivot center toward the center of spring force, when pinching the levers (i.e., finger grip portions 32, 34) toward each other to open for needle insertion. Figure 31 shows the needle depth stop 20 in the closed position, and Figure 32 shows the needle depth stop 20 in the open position (i.e., the finger grip portions 32, 34 being pinched together as represented by arrows 33). Dimension 300 in Figure 31 identifies the gap from the pin 52 and groove 54 pivot point 99 to the edge 97 of the spring 26 when the needle depth stop 20 closed. In Figure 32, dimension 302 identifies the gap from the pin 52 and groove 54 pivot point 99 to the edge 97 of the spring 26 when the needle depth stop 20 is open, and dimension 304 identifies the difference between gap 300 (i.e., when the needle depth stop 20 is closed) and gap 302 (i.e., when the needle depth stop 20 is open). As shown, the gap deceases when the needle depth stop 20 is opened. This dynamic shift of the pivot center toward the center of spring force results in a user actuation force that increases at a decreasing rate as the levers are pinched together. This location of the pivot does tend to cause the pivot connection to become less stable in use. Placing the pivot further from the center and closer to the circle reduces mechanical advantage but increases load on the pivot making the device more stable when clamped upon a needle. Preferably, the pivot location of the depth stop is about 38% of the circle radius away from center, as this location strikes a reasonable balance between opening force and pivot stability. One additional benefit derived by this mechanism is that the amount the "C" ring spring is spread when the device is opened to accept a needle is about 90% of the jaw separation required to insert a needle for use. This helps keep the spring expansion within the elastic and below the plastic ranges of the spring material.

Accommodation for a broad range of needles is achieved through a combination of the spring loaded jaw travel and a series of three decreasingly sized, opposed pairs of semicircular grooves 56, 58, 60, 62, 64, 66 separated by flats 71, 72, 73, 74 in the needle stop's jaw halves 36, 38. The resulting diameter these grooves describe when the jaws 36, 38 are aligned parallel to one another is slightly larger than the smallest needle intended to be clamped within that groove. Groove sizing done in this manner assures that the clamping jaws 36, 38 open ever wider to accommodate larger needles within that groove. This results in increased clamping force within a groove with each step up in needle diameter. The smallest grooves 56, 58 of the depth stop 20 may be set at .012" radius, sized to accommodate 25 gauge (.01125" radius) to 22 gauge (.013" radius) needles. The middle grooves 60, 62 may be set at .019" radius, sized to accommodate 21 gauge (.032") to 19 gauge (.042") needles. The largest grooves 64, 66 may be set at .030" radius, sized to accommodate 18 gauge (.057") to 16 gauge (.065") needles. Preferably, the largest grooves 64, 66 are situated closest to the jaw opening 68 to reduce the degrees of jaw opening necessary for loading.

Assembly of the device 20 requires that the mating pivot features of both jaws 36, 38 be engaged. The spring "C" clip 26 is then slid into place from the rear (finger grip area) of the device 20 just as a "C" clip 26 is installed on a grooved shaft. While the pivot features (i.e., the pin 52 and groove 54 configuration) hold the two body portions 22, 24 in fore/aft alignment, the "C" clip 26 serves to maintain registration of the two device halves and keep them aligned axially somewhat like a Chinese puzzle. Preferably, no other engagement details are required make the assembly stable.

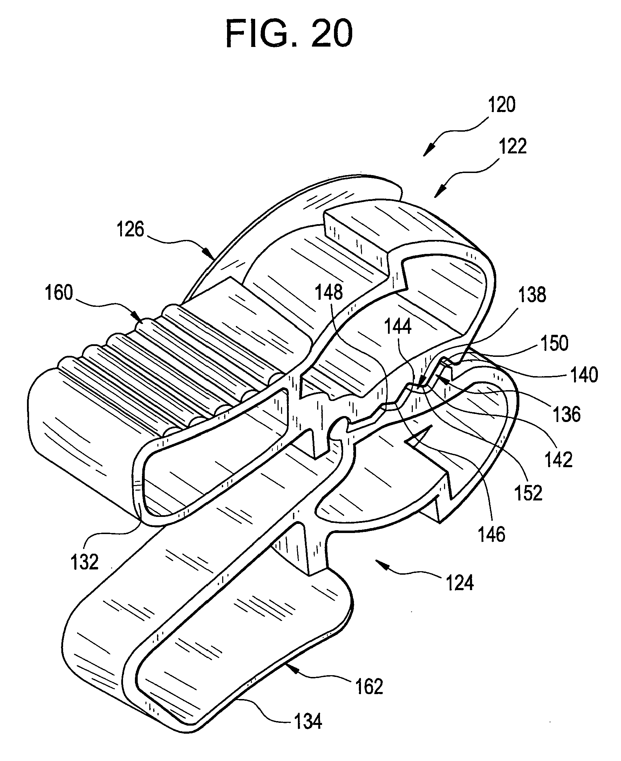

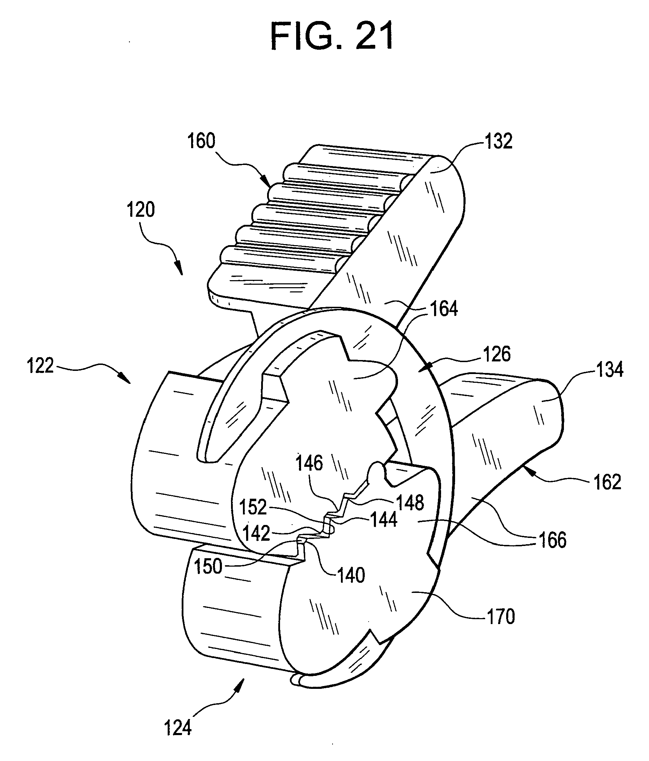

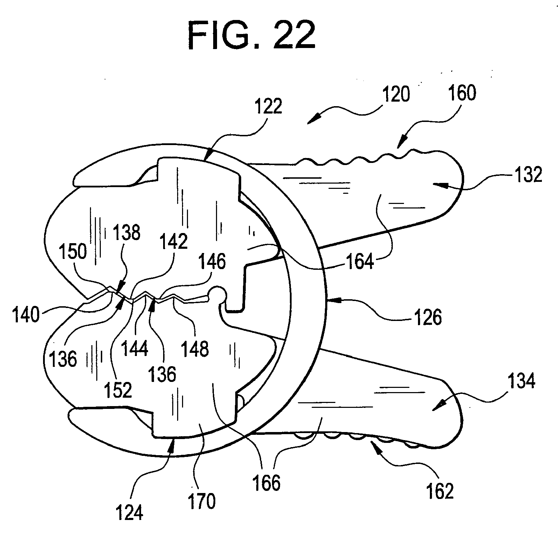

Figures 20-22 illustrate a needle depth stop 120 which is in accordance with a preferred embodiment of the present invention. The needle depth stop 120 shown in Figures 20-22 is very similar to the needle depth stop 20 and includes two body portions 122, 124 which are indexed together and are pivotable relative to each other due to a spring member 126 (which is much like spring member 26) which engages the two body portions 122, 124 and biases them together. As such, only the differences between the needle depth stop 120 and needle depth stop 20 will be explained in detail.

It has been found through user experiments with the needle depth stop 20 shown in Figures 4-17 that, although the gripping jaw configuration works as intended (i.e., the jaws 36, 38 do grip needles), some users have trouble selecting the best retention grooves for the needle they plan to use on their first attempt. In other words, users do not instinctively know which set of grooves to have hold a needle - i.e., grooves 56, 58, grooves 60, 62, or grooves 64, 66. Specifically, it has been found that sometimes users initially try to put a smaller needle in a larger groove. This has been determined to be due most often to the user's failure to open the jaws 36, 38 wide enough to allow needles to pass sufficiently deep within the device 20 for placement in the appropriate groove.

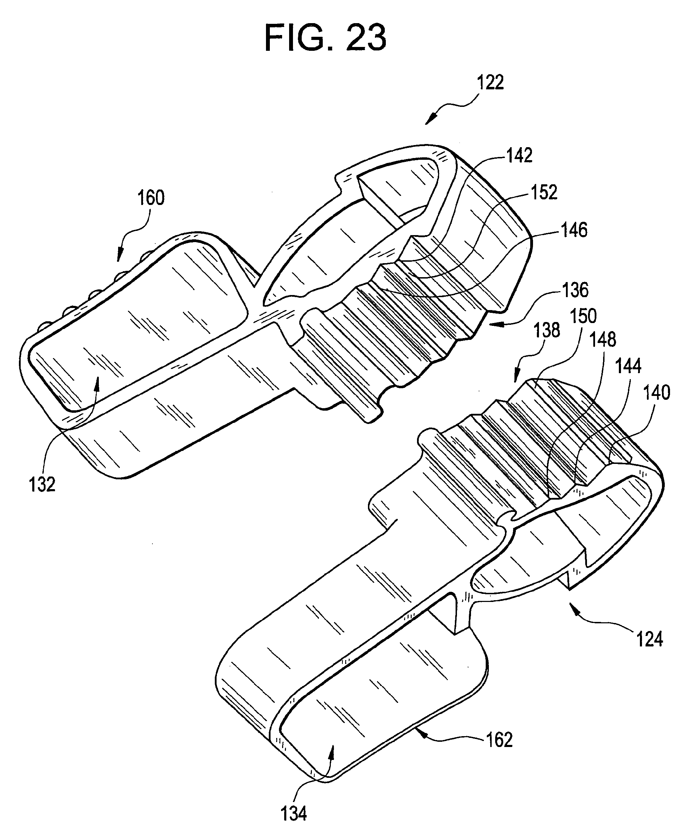

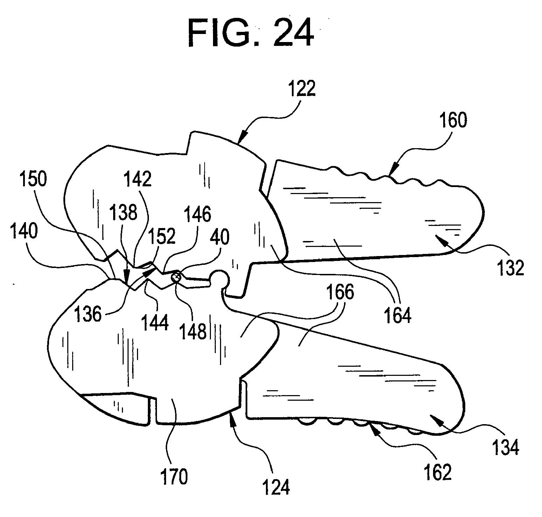

The needle depth stop 120 shown in Figures 20-22 has improved gripping jaw surfaces 136, 138 which are configured to make the device 120 easy and fairly transparent to the user. The gripping jaw surfaces 136, 138 make needle insertion difficult to get wrong. Figure 23 provides an exploded perspective view of the needle depth stop 120, with the spring member 126 omitted for clarity, and each of Figures 24-30 show the needle depth stop 120 gripping a different-sized Gauge needle. As shown in Figures 20-30, the gripper jaw surfaces 136, 138 are formed of a series of interlocking graduated serrations or teeth 140, 142, 144, 146, 148. By this method, the jaw spread which is required to receive a given needle diameter is reduced, and it is no longer possible to place a needle into a location where the needle cannot be gripped. If a stronger grip is desired, the user can easily place the needle deeper within the jaws 136, 138, into the next serration set.

The needle depth stop 120 shown in Figures 20-30 provides that five gripping locations (i.e., one for each tooth 140, 142, 144, 146, 148) are now available instead of the original three (as provided on the needle depth stop 20 shown in Figures 4-17), and every location is configured in a manner such that any needle within the intended use range (22-16 Gauge) will find a workable position on the first try.

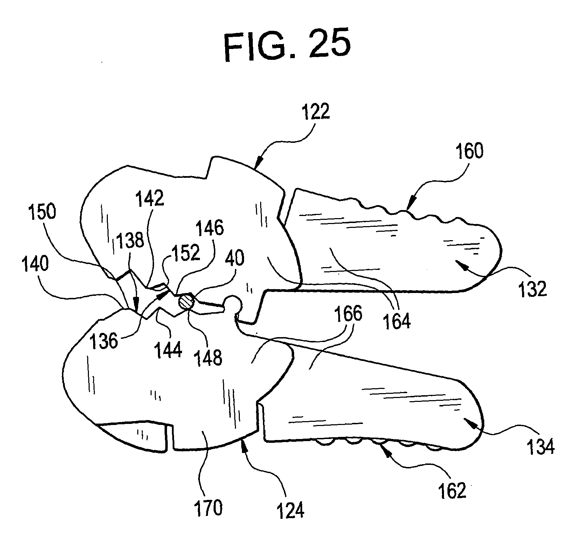

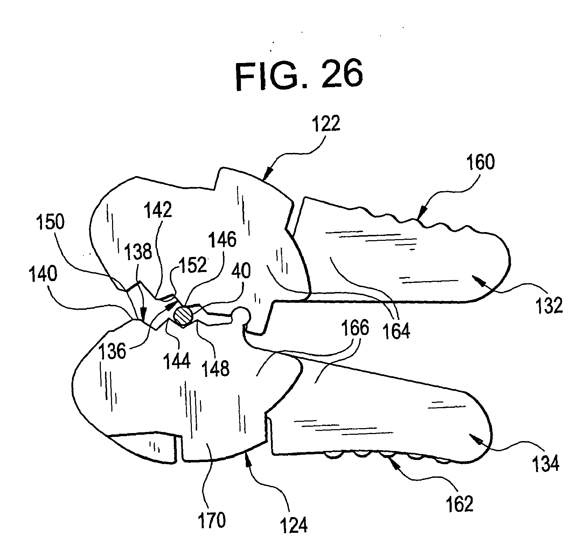

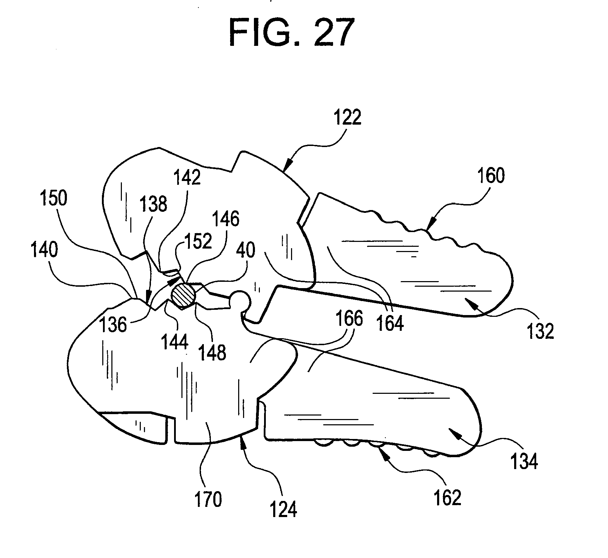

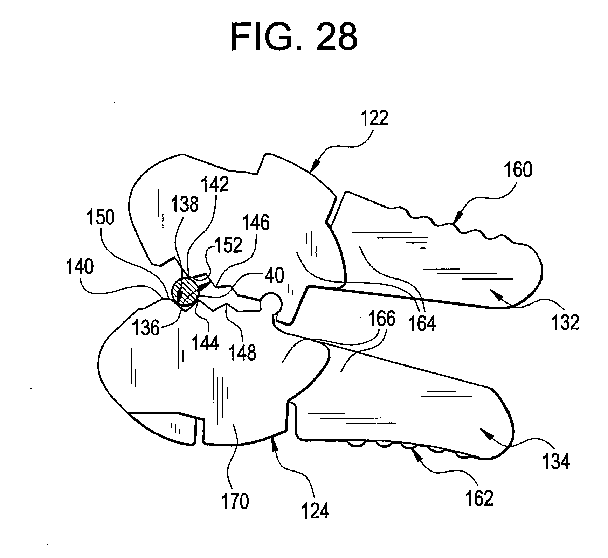

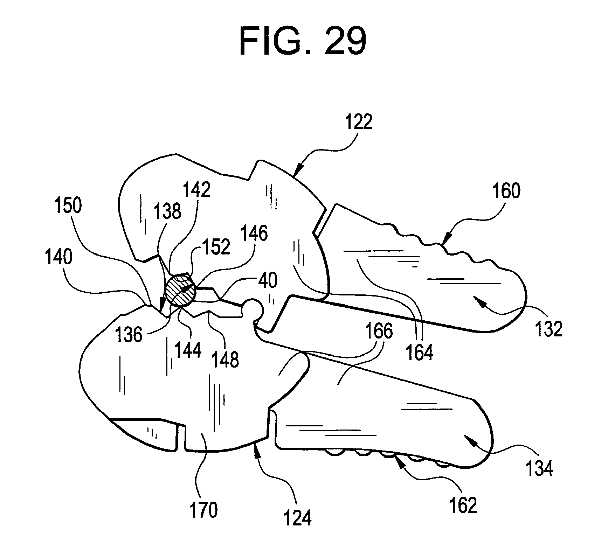

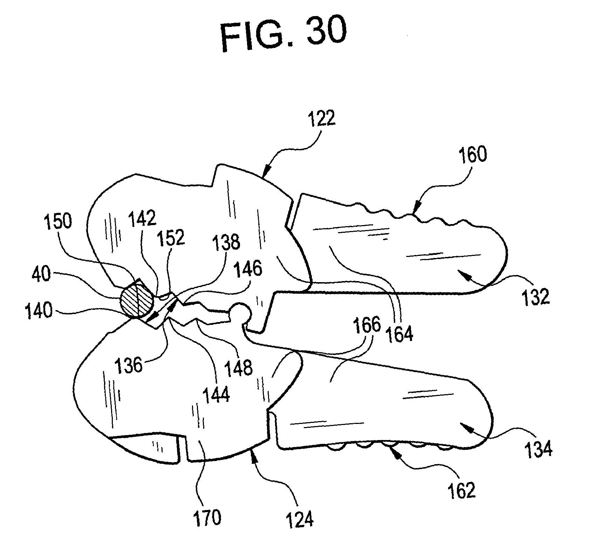

Each of Figures 24-30 shows the jaws 136, 138 of the needle depth stop 120 of Figures 20-22 gripping a different-sized Gauge needle. Specifically, Figure 24 shows the needle depth stop 120 gripping a 22 Gauge needle; Figure 25 shows the needle depth stop 120 gripping a 21 Gauge needle; Figure 26 shows the needle depth stop 120 gripping a 20 Gauge needle; Figure 27 shows the needle depth stop 120 gripping a 19 Gauge needle; Figure 28 shows the needle depth stop 120 gripping a 18 Gauge needle; Figure 29 shows the needle depth stop 120 gripping a 17 Gauge needle; and Figure 30 shows the needle depth stop 120 gripping a 16 Gauge needle.

As shown in Figures 20-30, the larger peaks of the serrations (i.e., serrations 140 and 142) have a flat 150, 152 provided on their crests. These flats 150, 152 spread the load surface of that serration or tooth 140, 142 upon a retained needle 40 and reduce required jaw spread to load a needle which subsequently helps keep expansion of the spring member within its elastic range and eases the user's efforts as well. As shown in Figures 28 and 30, the flats 150, 152 assure a good "three point" contact with the needle 40 while spreading the load over a bit more surface area without risk of excessive local deformation of larger retained needles. That would be particularly significant when thin wall needles are being used.

Other refinements with regard to the needle depth stop 120 shown in Figures 20-22 include a more ergonomic set of surfaces 160, 162 for user fingers to grasp and manipulate the device 120. Also, the central molded wall 164, 166 of each of the parts 122, 124 has been moved out to the face 170 of the device 120 opposite the finger grips 132, 134 so when placed on a needle 40, a nice flat face 170 is provided at the depth stopping surface.

It should be noted that, with either needle depth stop 20 or 120, the location at which the spring 26, 126 delivers its force provides some distinct advantages. Because the device 20, 120 utilizes a pivot that is not locked together such as a traditional pinned hinge, the forces delivered by the spring 26, 126 at the spring tips bear most strongly upon the larger needles placed closest to the open end of the jaw and in a more direct line with where the spring delivers its force. Deeper towards the pivot where small needles would be placed, there is actually less load because the hinge members are not locked together. Larger needles require more holding force than do smaller needles so the action of the device delivers its load in accordance with what is required. If the pivot were locked together, the opposite would be true and a crushing load could be delivered to small needles.

While embodiments of the present invention are shown and described, it is envisioned that those skilled in the art may devise various modifications of the present invention without departing from the spirit and scope of the present disclosure.

## Claims

1. A needle depth stop (20, 120) **characterized by** a plurality of body portions (22, 24, 122, 124) which are indexed together, pivotable relative to each other, and are spring biased together, wherein the body portions (22, 24, 122, 124) comprise finger grip portions (32, 34, 132, 134) which are pinchable together to open the needle depth stop (20, 120), and wherein the body portions (22, 24, 122, 124) comprise jaws (36, 38, 136, 138) which are configured to grip a needle (40) when the needle depth stop (20, 120) is biased closed.

2. A needle depth stop (20, 120) as recited in claim 1, further **characterized by** a spring member (26, 126) which engages the body portions (22, 24, 122, 124) and spring biases the needle depth stop closed (20, 120).

3. A needle depth stop (20, 120) as recited in claim 1, **characterized in that** the needle depth stop (20, 120) is configured such that a translation of the pivot point (99) toward the center of spring load as the jaws (36, 38, 136, 138) open, results in the jaws opening force increasing at a decreasing rate.

4. A needle depth stop (20, 120) as recited in claim 2, **characterized in that** the spring member (26, 126) comprises a C ring.

5. A needle depth stop (20, 120) as recited in claim 2, **characterized in that** the needle depth stop (20, 120) is configured such that the spring member (26, 126) registers the body portions (22, 24, 122, 124) in alignment with one another.

6. A needle depth stop (20, 120) as recited in claim 1, **characterized in that** the jaws (36, 38) comprise a plurality of adjacent grooves (56, 58, 60, 62, 64, 66), wherein each groove (56, 58, 60, 62, 64, 66) is configured to accommodate a different range of needles.

7. A needle depth stop (20, 120) as recited in claim 1, further **characterized by** a flat (71, 72, 73, 74, 150, 152) being disposed between adjacent grooves (56, 58, 60, 62, 64, 66).

8. A needle depth stop (20, 120) as recited in claim 6, **characterized in that** the grooves (56, 58, 60, 62, 64, 66) of each jaw (36, 38) are paired and each of the groove pairs (56, 58, 60, 62, 64, 66) is of different size with the largest groove pair (64, 66) situated closest to a jaw opening (68) of the needle depth stop (20).

9. A needle depth stop (20, 120) as recited in claim 1, **characterized in that** the needle depth stop (20, 120) is configured such that the body portions (22, 24, 122, 124) are affixed to each other by a spring member (26, 126).

10. A needle depth stop (20, 120) as recited in claim 1, **characterized in that** the body portions (22, 24, 122, 124) are indexed together via an unlinked hinge.

11. A needle depth stop (20, 120) as recited in claim 10, **characterized in that** the unlinked hinge comprises a pin and groove configuration (52, 54).

12. A needle depth stop (20, 120) as recited in claim 10, **characterized in that** the hinge indexing the body portions (22, 24, 122, 124) together is free to separate when needles are inserted into the jaw opening portion closest to the hinge.

13. A needle depth stop (20, 120) as recited in claim 1, **characterized in that** each body portion (22, 24, 122, 124) has an outer surface (42, 44), and the outer surface (42, 44) of each body portion (22, 24, 122, 124) includes a recess (46, 48) which defines a groove (47, 49), a step (55, 57) and a pocket (59, 61).

14. A needle depth stop (20, 120) as recited in claim 1, further **characterized by** a C ring (26, 126) which has ends (50, 51) and engages the body portions (22, 24, 122, 124), wherein the C ring (26, 126) biases the needle depth stop (20, 120) closed, wherein each body portion (22, 24, 122, 124) has an outer surface (42, 44), and the outer surface (42, 44) of each body portion (22, 24, 122, 124) includes a recess (46, 48) which defines a groove (47, 49), a step (55, 57) and a pocket (59, 61), wherein each of the grooves (47, 49) receives a portion of the C ring (26, 126), proximate the ends (50, 51) of the C ring (26, 126), and each of the pockets (59, 61) receives a corresponding projection (70) at the end (50, 51) of the C ring (26, 126).

15. A needle depth stop (20, 120) as recited in claim 1, **characterized in that** the needle depth stop (20, 120) is side loading, wherein a needle (40) is loadable from a side of the needle depth stop (20, 120), past an opening (68), in between the set of jaws (36, 38, 136, 138).

16. A needle depth stop (120) as recited in claim 6, **characterized in that** the grooves comprise a plurality of adjacent "V"-shaped valleys arranged as jaw serrations (140, 142, 144, 146, 148).

17. A needle depth stop (120) as recited in claim 16, **characterized in that** at least one of the serrations (140, 142, 144, 146, 148) includes a flat (150, 152).

18. A needle depth stop (120) as recited in claim 17, **characterized in that** the flat (150, 152) is configured to provide that the needle depth stop (120) grips a needle (40) at three points.

19. A needle depth stop (120) as recited in claim 16, **characterized in that** the serrations (140, 142, 144, 146, 148) are configured to provide that the jaws (36, 38, 136, 138) have at least five needle-gripping locations.

20. A needle depth stop (120) as recited in claim 16, **characterized in that** the jaw serrations (36, 38, 136, 138) are of increasing size, the largest serration (140) being situated closest to a jaw opening of the needle depth stop.

21. A needle depth stop (120) as recited in claim 16, **characterized in that** opposing jaw serrations (140, 142, 144, 146, 148) intermesh with one another.

22. A needle depth stop (120) as recited in claim 1, **characterized in that** the body portions (122, 124) are configured to provide that the needle depth stop (120) has a flat face (164, 166).
